# EUROPEAN PATENT SPECIFICATION

(11) **EP 2 975 005 B1**
(45) Date of publication and mention of the grant of the patent: **28.07.2021**
(21) Application number: 13878210.7
(22) Date of filing: 13.03.2013
(51) Int. Cl.: C02F 3/00, B09B 3/00, B09B 5/00, C02F 3/34, C12N 1/20, C12N 1/00, C12N 3/00

(54) **METHOD FOR PRODUCING A SEEDING AGENT**
VERFAHREN ZUR HERSTELLUNG EINES SAATMITTELS
PROCÉDÉ DE PRODUCTION D'UN AGENT D'ENSEMENCEMENT

(43) Date of publication of application: 20.01.2016
(73) Proprietor: Citic Co., Ltd., Hidaka-gun, Hokkaido 056-0014 (JP)
(72) Inventor: NAMIKATA, Masahiro, Hidaka-gun Hokkaido 056-0014 (JP); TAKEBE, Fumihiko, Hidaka-gun Hokkaido 056-0014 (JP)
(74) Representative: EIP
(86) International application number: PCT/JP2013/057017
(87) International publication number: WO 2014/141406

(56) References cited:
- JP-A- H0 912 387
- JP-A- H0 912 387
- JP-A- H04 119 984
- JP-A- H04 119 984
- JP-A- H06 144 976
- JP-A- H06 144 976
- JP-A- H07 232 985
- JP-A- H09 227 260
- JP-A- 2000 102 378
- JP-A- 2003 334 573
- JP-A- 2005 041 738
- JP-A- 2005 041 738
- JP-A- 2006 198 609
- JP-A- 2006 198 609
- JP-A- 2008 189 820
- JP-A- 2010 119 937
- None

## Description

### TECHNICAL FIELD

The present invention relates to the field of wastewater treatment, and more specifically to a method of producing a seeding agent for effective start-up or adjustment of a function of a wastewater treatment facility.

### BACKGROUND ART

In a facility for performing wastewater treatment by means of a microorganism, such as a septic tank, a sewerage system or a rural community wastewater treatment plant, depending on adequacy of an operational state during functional start-up or functional adjustment of the facility, subsequent wastewater treatability will greatly vary. "Seeding of a microorganism (hereinafter also referred to simply as "seeding")" during the functional start-up or functional adjustment is important for enhancing the wastewater treatability. Heretofore, seeding has been performed based on a method comprising: withdrawing a certain amount of sludge from an existing facility such as a night-soil treatment plant or a sewage treatment plant; and putting, as seed sludge, the withdrawn sludge into a treatment tank of a target facility to be subjected to functional start-up or functional adjustment, or putting, into the treatment tank, a soil bacteria-containing supernatant solution obtained by suspending soil in water. Failing to use such seed sludge causes a problem that, in many cased, it needs to take several weeks to several months before completion of the functional start-up or functional adjustment, and wastewater treatability cannot be achieved at a required level or cannot be improved.

However, the conventional seeding method requires the steps of: selecting a facility capable of offering sludge suitably usable in a target facility to be subjected to functional start-up or functional adjustment, among a large number of existing facilities operated in various states; withdrawing sludge from the selected facility at an adequate timing; and putting, as seed sludge, the withdrawn sludge into the target facility. Thus, the overall time, effort and cost required for the work operation will be significantly increased. Moreover, the method comprising putting the supernatant solution into the treatment tank is liable to cause instability in effect of seeding.

With a view to solving these problems, an agent for seeding (hereinafter referred to as "seeding agent") is offered commercially. This seeding agent is being increasingly used in such a manner as to be put into a septic tank during functional start-up or functional adjustment thereof in order to achieve early establishment of wastewater treatability and enhancement in wastewater treatability. As a commercially-available seeding agent, there has been known a seeding agent formulated using a specific, single type of aseptically cultured microorganism by itself, or using a mixture of several types of microorganisms each aseptically cultured.

The inventers of the present invention have also already proposed seeding agent production methods disclosed in the following Patent Documents 1 and 2.

The Patent Document 1 proposes a method for producing a seeding agent using livestock excrement as a raw material. Specifically, as the method for producing a seeding agent using livestock excrement as a raw material, the Patent Document 1 describes a technique of: mixing a raw material containing swage sludge, livestock excrement, seed bacteria, minnows and a moisture control agent at respective give rates; fermenting the resulting raw material while adjusting an amount of air to be supplied to the raw material (air supply amount to the raw material) in two stages; and then maturing the fermented raw material under the condition that the air supply is completely stopped.

The Patent Document 2 proposes a method for compacting, into a block, a seeding agent prepared by mixing livestock excrement and a moisture control agent together and aerobically fermenting the resulting mixture. The Patent Document 2 describes a technique of compacting, into a block, a seeding agent produced, for example, by the technique described in the Patent Document 1 to thereby extend a residence time of the seeding agent in a wastewater treatment facility and thus facilitate effective utilization of the seeding agent.

The following Patent Document 3 proposes a seeding agent production method comprising: mixing a raw material seeding agent containing sewage sludge and animal and plant residues, and wood chips; and fermenting the resulting mixture. The Patent Document 3 describes a technique of: sieving a mixture after fermentation; and observing a cross-section of a deposit of a sieved fraction to determine the progress of the fermentation to suppress quality variation.

Meanwhile, in recent years, introducing possibly many types of microorganisms into a wastewater treatment system has been increasingly deemed as an ideal seeding method. For realizing such a method, there has been proposed a constructed wetland wastewater treatment system utilizing a complex microbial system comprising a variety of soil microorganisms.

Soil contains an abundance of microorganisms useful for environmental cleanup, and, in particular, contains gram-positive bacteria in a large amount. Examples of the gram-positive bacteria include Bacillus bacteria to which natto bacteria belong, Clostridium bacteria, lactic bacteria, and actinomycetes. It is known that these bacteria effectively function for wastewater treatment, and examples of their advantageous effect include: high decomposition ability against organic pollutants; promotion of nitrogen removal; deodorizing effect, enhancement in treatment efficiency; and effect of suppressing the occurrence of excess sludge. These bacteria are highly resistant to environmental stress, and thus advantageously hard to become extinct.

Therefore, there is a need for a seeding agent containing a variety of effective microorganisms such as a variety of gram-positive bacteria to allow a seeding operation to be conveniently performed during functional start-up or functional adjustment of a wastewater treatment facility.

### CITATION LIST

### [Parent Document]

Patent Document 1: JP 4325980B
Patent Document 2: JP 4627718B (equivalent to JP 2006-198609A)
Patent Document 3: JP 2007-209933A
Patent Document 4: JP 4627718B

### SUMMARY OF INVENTION

### [Technical Problem]

Considering the above viewpoint, the conventional techniques have the following problems.

The conventional commercially-available seeding agent contains a few types of effective microorganisms, and thus has low biodiversity, thereby failing to be utilized in each of a plurality of wastewater treatment facilities placed in various treatment environments, in a versatile manner. Moreover, the seeding agent containing an aseptically-cultured effective microorganism is high in cost, and thus often used only in a limited application, in existing situations.

Although the technique described in the Patent 1 relates to a seeding agent production method, its object at the time of filing of the application is focused mainly on reutilizing livestock excrement which has heretofore been discarded as industrial waste at enormous costs. Therefore, the Patent Document 1 does not make any mention of a type of bacterium contained in the seeding agent, and biodiversity of the seeding agent. That is, the technique described in the Patent Document is not based sufficient studies on a production process for obtaining a seeding agent containing a variety of and a large amount of effective microorganisms capable of effectively functioning in wastewater treatment facilities placed in various treatment environments. This also applies to the techniques described in the Patent Documents 2 and 3.

Therefore, regarding a seeding agent capable of being conveniently put into each of a plurality of wastewater treatment facilities placed in various treatment environments in order to bring out a required treatability within a short period of time, during functional start-up or functional adjustment of the wastewater treatment facility, it is an object of the present invention to provide a method for producing a seeding agent containing a variety of and a large amount of effective microorganisms, particularly, gram-positive bacteria, from a raw material containing organic waste, at low cost.

### [Solution to Technical Problem]

Based on a finding that a seeding agent containing a variety of and a large amount of gram-positive bacteria as effective microorganisms capable of effectively functioning in wastewater treatment facilities can be obtained by improving a step of drying an organic waste-containing raw material after fermenting the raw material, the inventors have accomplished the present invention

According to a first aspect of the present invention, there is provided a method for producing a seeding agent. This method comprises a fermentation step, a first drying substep and a second drying substep. The fermentation step is a process of aerobically fermenting a raw material comprising organic waste and an auxiliary material. The organic waste may be one selected from the group consisting of sewage sludge, livestock excrement, and food waste, or a combination of two or more thereof. The first drying substep is a process of drying the fermented raw material after being subjected to the fermentation step, until a water content thereof becomes a value falling within a first range which is less than that as measured just after completion of the fermentation step. The first range is from 40% to 55%. More preferably, a water content of the raw material as measured just after completion of the first drying substep is preferably 50% or less. The second drying substep is a process of further drying the dried raw material after being subjected to the first drying substep, until a water content thereof becomes a value falling within a second range which is less than a lower limit of the first range, within a period of time which is less than a period of time required for the first drying substep. The second range is from 10% to 30%. More preferably, a water content of the raw material as measured just after completion of the second drying substep is preferably 20% or less.

As above, the drying step is performed in two stages: the first drying substep configured to gradually dry the fermented raw material by taking a relatively long period of time; and the second drying substep configured to rapidly dry the dried raw material after being subjected to the first drying substep, within a relatively short period of time. Thus, in the first drying substep, microorganisms contained the fermented raw material are partially sporulated, and, in the second drying substep, microorganisms other than the microorganisms sporulated in the first drying substep are decomposed, so that it becomes possible to produce a seeding agent containing a variety of and a large amount of effective microorganisms including Bacillus bacteria, as microorganisms capable of effectively functioning in wastewater treatment facilities.

In one preferred embodiment, the method further comprises a step of, before the second drying substep, removing a remaining part of the auxiliary material from the raw material after being subjected to the first drying substep.

In another preferred embodiment, the fermentation step comprises a first fermentation substep and a second fermentation substep. The first fermentation substep is a process of increasing a fermentation temperature of the raw material to a temperature of 70°C or more, at least two times. The second fermentation substep is a process of increasing a fermentation temperature of the raw material after being subjected to the first fermentation substep, to a temperature of 50°C or more, at least one time.

More preferably, at least the second fermentation substep includes a sub-substep of plowing the raw material, wherein, when the temperature of the raw material in the sub-substep of plowing does not rise beyond 50°C any more, the fermentation step is terminated.

Also disclosed in the present application is a block-shaped seeding agent which is obtained by compacting, into a block, a seeding agent produced by the above method (recited in any one of the appended claims 1 to 9).

The present invention makes it possible to produce a seeding agent capable of, when it is put into a treatment tank during functional start-up or functional adjustment of each of a plurality of wastewater treatment facilities placed under different treatment conditions, completing the functional start-up or functional adjustment of the wastewater treatment facility within a short period of time, irrespective of difference in treatment conditions, while achieving a required and stable treatability.

### BRIEF DESCRIPTION OF DRAWINGS

FIG. 1 is a flow chart illustrating steps of a seeding agent production method according to one embodiment of the present invention.
FIG. 2 presents a transition of a fermentation temperature in the seeding agent production method according to this embodiment.
FIG. 3 presents a result obtained by analyzing a type of microorganism contained in a seeding agent produced by the seeding agent production method according to this embodiment.
FIG. 4A illustrates an analysis result showing that a type of bacterium emerging when using a seeding agent varies depending on a drying step, wherein FIG. 4A presents a type of bacterium emerging when using, as the seeding agent, a sample A obtained at a time of completion of fermentation.
FIG. 4B illustrates an analysis result showing that a type of bacterium emerging when using a seeding agent varies depending on a drying step, wherein FIG. 4B presents a type of bacterium emerging when using, as the seeding agent, a sample B prepared by drying the sample A obtained at the time of completion of fermentation, until a water content thereof becomes 50% or less, by taking about 2 weeks.
FIG. 4C illustrates an analysis result showing that a type of bacterium emerging when using a seeding agent varies depending on a drying step, wherein FIG. 4C presents a type of bacterium emerging when using, as the seeding agent, a sample C prepared by drying the sample A obtained at the time of completion of fermentation, until a water content thereof becomes 20% or less, by taking 24 hours.
FIG. 4D illustrates an analysis result showing that a type of bacterium emerging when using a seeding agent varies depending on a drying step, wherein FIG. 4D presents a type of bacterium emerging when using, as the seeding agent, a sample D prepared by drying the sample B until a water content thereof becomes 20% or less, at 50°C by taking 24 hours.
FIG. 5 is a photograph showing microbial biodiversity of a seeding agent produced by the seeding agent production method according to this embodiment.

### DESCRIPTION OF EMBODIMENTS

The present invention, which will now be described in detail, relates to a method in accordance with claim 1.
Dependent claims refer to preferred embodiments of the invention.

A seeding agent production method according to the present invention is characterized in that a drying step of drying a raw material after being subjected to a fermentation step is performed in two stages: a first drying substep of gradually drying the raw material; and a second drying substep of rapidly drying the raw material, so that it becomes possible to allow a seeding agent produced by the production method to contain, as effective microorganisms, a variety of and a large amount of gram-positive bacteria including Bacillus bacteria, Clostridium bacteria, lactic bacteria and actinomycetes, as compared to seeding agents produced by the conventional methods. For example, a seeding agent production method according to one embodiment of the present invention can produce a seeding agent in which gram-positive bacteria account for at least 70% or more of all bacteria contained therein. In one preferred embodiment of the present invention, a seeding agent may be produced as dried pellets each having a water content of about 10 to 30 weight%. Alternatively, in another preferred embodiment of the present invention, a seeding agent may be produced as a block-shaped body or a creamlike or jellylike semisolid body formed using the pellets, or a liquid body obtained by mixing the pellets in a certain liquid.

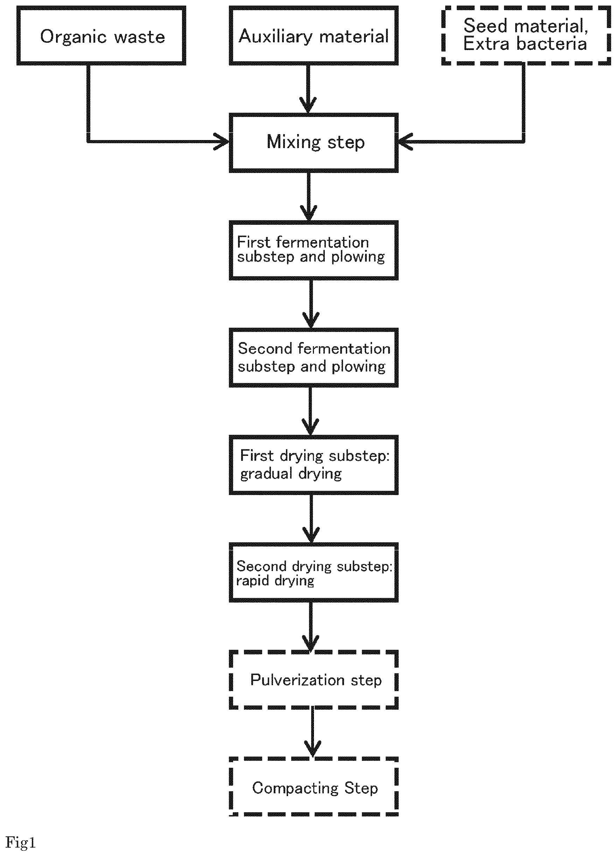

A seeding agent production method according to one embodiment of the present invention will be described below. FIG. 1 is a flow chart illustrating steps of the seeding agent production method according to this embodiment.

### [Raw Materials]

In the production method according to this embodiment, organic waste and an auxiliary material are used as a raw material. As the organic waste, it is possible to use, for example, sewage sludge, livestock excrement, and food waste. As livestock excrement, it is preferable to use cow dung with straw pieces. The use of such cow dung makes it possible to cause Bacillus bacteria to be mixed in a produced seeding agent in a large amount.

The auxiliary material is a material for use as a host material, a moisture adjustment material and a density adjustment material, and it is possible to use, for example, wood chips, rice straw pieces, and sawdust. The wood chips are preferably softwood chips. The softwood chips are particularly preferable as the auxiliary material, because they are not easily decomposed, thereby being capable of withstanding repetitive use, and contain a large amount of a resin component, thereby being capable of producing a large amount of metabolite having a pathogen killing effect, through microbial metabolism during the fermentation step.

With a view to accelerating the production of a seeding agent, and providing a finally-obtained seeding agent with higher homogeneity, seed compost prepared by fermenting compost, or a commercially-available microbial formulation, may be optionally added as a seed material to the raw material. Further, in the case where it is necessary to add a specific function to a finally-obtained seeding agent, specific bacteria or soil containing the specific bacteria may be optionally added to the raw material according to the specific function.

### [Mixing Step]

The organic waste and the auxiliary material, and the optional seed material, specific bacteria and/or specific bacteria-containing soil, are homogeneously mixed together. As regards an amount of each component contained in the raw material, the organic waste is preferably contained in an amount of 20 to 80 weight%, and the auxiliary material is preferably contained in an amount of 20 to 45 weight%. Preferably, the optional seed material, specific bacteria and/or specific bacteria-containing soil are contained in an amount up to 10 weight%. Preferably, a water content of the raw material is adjusted in such a manner as to fall within a certain range. The water content of the raw material can be adjusted by changing the amount of the auxiliary material to be contained in the raw material, and is preferably set to about 60 to 70 weight%, more preferably, about 65 to 69 weight%. Preferably, the water content of the raw material is changed depending on whether the raw material is used in winter or in summer, wherein, in summer, the water content is set to a higher value. A bulk specific gravity of the mixture can be adjusted by the amount of the auxiliary material, and is preferably set to about 0.5 to 0.6. When the raw material is used in winter, the bulk specific gravity is preferably set to a relatively high value.

The raw material is homogeneously mixed under stirring, for example, using a manual spreader. Preferably, in the mixing, the stirring is performed after stacking up the auxiliary material and the organic waste in this order. The homogeneously-mixed raw material in this manner is put into a fermenter.

### [Fermentation Steps]

Then, the homogeneously-mixed raw material is loaded into the fermenter and subjected to fermentation. In the present invention, the raw material is subjected to aerobic fermentation while an air supply amount to the raw material is controlled such that a temperature of the raw material rises to or beyond about 70°C two times or more, and rises to or beyond about 50°C one time or more. As regards the temperature of the raw material, respective temperatures of several depth positions apart from a surface of the loaded raw material by 1 m or less are measured, and a highest one of the measured temperatures may be defined as the temperature of the raw material in the fermentation step.

Specifically, the raw material is loaded into the fermenter, and fermented until the temperature thereof rises to or beyond about 70°C under control of the air supply amount to the raw material. The control of the air supply amount, i.e., increasing and reducing of the air supply amount, may be performed by increasing and reducing an air supply amount per unit time, or by increasing and reducing an air supply time while maintaining the air supply amount per unit time. Subsequently, when the temperature of the raw material falls below 70°C, or when a given period of time (e.g., about 3 to 14 days depending on conditions such as temperature and humidity) has elapsed under the condition that the temperature of the raw material is maintained at a temperature of about 70°C or more, plowing may be performed. As used herein, the term "plowing" means an operation of stirring the raw material to supply oxygen to the entire raw material to thereby homogenously promote the fermentation, and suppress excess formation of an aggregated structure to thereby suppress excessive temperature rise to prevent carbonization of the raw material. After the plowing, under control of the air supply amount, the fermentation is promoted until the temperature of the raw material rises to or beyond about 70°C again, and then next plowing is performed at the same timing as that mentioned above. The process of fermenting the raw material under the condition that the temperature of the raw material rises to or beyond about 70°C at least two times or more, by controlling the air supply amount and performing plowing is a first fermentation substep.

A number of times the temperature of the raw material is increased to about 70°C or more, in the first fermentation substep, is appropriately determined while taking into account quality of a finally-obtained seeding agent and a permissible production lead time. The first fermentation substep is terminated when the temperature of the raw material does not rise beyond 70°C any more, or when plowing is performed in response to an elapse of a given period of time (e.g., about 3 to 14 days) under the condition that the temperature of the raw material is maintained at a temperature of about 70°C or more.

After the first fermentation substep, the temperature of the raw material is lowered by controlling the air supply amount (specifically, by reducing the air supply amount). Then, when the temperature of the raw material falls below about 50°C, or when a given period of time (e.g., about 5 to 14 days depending on conditions such as temperature and humidity) has elapsed after the temperature of the raw material does not rise beyond 70°C, plowing is performed. After the plowing, under control of the air supply amount, the raw material is further fermented for a given period of time (e.g., about 5 to 14 days depending on conditions such as temperature and humidity) under the condition that the temperature of the raw material is set to a temperature of about 50°C or more. This process is a second fermentation substep. The second fermentation substep is terminated when the temperature of the raw material rises to or beyond about 50°C at least one time, and this state is maintained for a given period of time (about 5 to 10 days), or when the temperature of the raw material does not rise beyond about 50°C even after repeatedly performing plowing. The water content of the raw material as measured just after completion of the fermentation step is generally about 55 to 60%.

A number of times the temperature of the raw material is increased to a temperature of about 50°C or more, in the second fermentation substep, is appropriately determined while taking into account quality of a finally-obtained seeding agent and a permissible production lead time. As the number of times the temperature of the raw material is increased to a temperature of about 50°C or more becomes larger, bacteria capable of growing in a normal temperature range become more dominant during use of a finally-obtained seeding agent.

As above, the fermentation step is performed under control of a target temperature during the fermentation and the number of times reaching the target temperature. This provides the following advantageous effects.

### (1) Decomposition and removal of easily-decomposable excess organic matter

Excess organic matter can be decomposed and removed to achieve stabilization of the seeding agent, and prevent an excessive increase in environmental load even if the seeding agent is put into a wastewater treatment facility.

### (2) Proliferation of various microorganisms associated with decomposition of organic matter

A variety of microorganisms proliferate in response to changes in environmental conditions during the fermentation, so that it becomes possible to additionally impart, to the seeding agent, microbial biodiversity and thus functionality compatible with wide wastewater treatment conditions.

### (3) Removal of unwanted matters by increase in temperature

The fermentation temperature can be adequately increased to remove (e.g., annihilate) unwanted matters for the seeding agent, e.g., unwanted bacteria such as Escherichia coli, vegetable seeds, and bugs such as fly, to stabilize the seeding agent.

Preferably, the fermenter used in this embodiment is a type configured to supply air from a bottom thereof toward the raw material, e.g., configured such that an air blast pipe is buriedly inserted into a bottom wall thereof. The use of the fermenter configured as above makes it possible to adequately send air to an inside of the raw material. Air supply into the fermenter can be achieved, for example, by sending air to the air blast pipe provided in the bottom wall using a heretofore known air blower. More preferably, this type of fermenter is plurally provided, wherein plowing is performed by transferring the raw material from one of the fermenters to another one of the fermenters.

The fermented raw material after being subjected to the fermentation step is then dried until the water content thereof finally falls within the range of about 10 to 30%. The present invention is characterized in that a drying step of drying the raw material is performed in two stages: a first drying substep; and a second drying substep. The first drying substep is a process of gradually drying the fermented raw material until a water content thereof becomes a value falling within a given range which is less than that as measured just after completion of the fermentation step, and the second drying substep is a process of rapidly drying the dried raw material after being subjected to the first drying substep, until a water content thereof becomes a value falling within a given range which is less than that as measured just after completion of the first drying substep.

### [First Drying Substep]

The first drying substep is a process of gradually drying the fermented raw material after being subjected to the fermentation step, until a water content thereof becomes a value falling within a first range which is less than that as measured just after completion of the fermentation step, by taking a certain level of time. The first range of the water content of the fermented raw material is about 40 to 55%, preferably, about 40 to 50%. The term "gradually" means that a period of time necessary to dry the fermented raw material until the water content thereof becomes a value falling within the first range is greater than a period of time necessary for the second drying substep described in detail later, i.e., necessary to dry the raw material after being subjected to the first drying substep, until a water content thereof becomes a value falling within a second range which is less than a lower limit of the first range.

Preferably, in the first drying substep, the raw material is dried by supplying thereto air at normal temperature, while plowing is appropriately performed. Preferably, a number of times of plowing during the fermentation step is greater than a number of times of plowing during the fermentation step. In one preferred embodiment, plowing is performed one time or more per week for 2 weeks, and then continuously performed one time or more per day for 10 to 14 days. The air supply amount may be appropriately set depending on a state of the raw material. The first drying substep is preferably performed for 2 to 4 weeks.

The first drying substep may include natural drying such as sun drying, as necessary. For example, the natural drying may be performed by, every time plowing of the raw material is performed, extracting a part of the raw material to expose it to the sun, and then returning the dried raw material to the original raw material.

As above, the water content of the raw material after being subjected to the fermentation step is reduced by taking a relatively long period of time. This provides the following advantageous effects.

### (1) Facilitation of Sporulation

Drying by taking a relatively long period of time makes it possible to gradually apply stress to sporulating bacteria to stimulate sporulation, thereby allowing microorganism to stably exist in the seeding agent in the form of spore.

### (2) Imparting of Microbial Biodiversity

Gradual decrease in reaction speed makes it possible to prevent only a specific bacteria from proliferating to thereby achieve microbial biodiversity in the seeding agent.

### [Second Drying Substep]

The second drying substep is a process of rapidly drying the dried raw material after being subjected to the first drying substep, until a water content thereof becomes a value falling within a second range which is less than a lower limit of the first range. The second range of the water content of the dried raw material is about 10 to 30%, preferably, about 10 to 20%. The term "rapidly" means that a period of time necessary to dry the raw material after being subjected to the first drying substep, until the water content thereof becomes a value falling within the second range is less than the period of time necessary for the first drying substep, i.e., necessary to dry the raw material after being subjected to the fermentation step, until the water content thereof becomes a value falling within the first second range. The former period of time is preferably less than one-half of the latter period of time. Specifically, the former period of time is preferably 1 to 7 days, most preferably, 2 days or less.

Preferably, in the second drying substep, with a view to rapidly drying the raw material, the raw material after being subjected to the first drying substep is forcedly dried, for example, by using a hot air from a drying device. Preferably, in the second drying substep, the raw material is dried at a possibly low temperature. Thus, it is desirable to dry the raw material while maintaining the temperature of the raw material at a temperature less than 60°C, by adjusting the air supply temperature.

As above, the water content of the raw material after being subjected to the first drying substep is reduced within a relatively short period of time. This provides the following advantageous effects.

### (1) Enhancement in Storage Stability of Seeding Agent

A microbial reaction is stopped to decompose and degrade excess organic matter other than microorganisms sporulated in the first drying substep and prevent transition and mutation of further microbial flora to thereby enhance storage stability of the seeding agent.

### (2) Imparting of Starter Function

Microorganisms useful for wastewater treatment are dominantly contained, and microbial metabolite is left to create a highly reactive state, so that it becomes possible to impart a starter function to the seeding agent so as to facilitate proliferation of the microorganisms when used.

Preferably, the raw material after being subjected to the first drying substep is subjected to a sieving step, before the second drying substep. Generally, at a time when the first drying substep is completed, the auxiliary material in the raw material is fully decomposed. However, depending on a type or state of the auxiliary material, there is a possibility that a part of the auxiliary material remains in the seeding agent without being decomposed at that time. Such a seeding agent is likely to exert a negative influence on a wastewater treatment facility when it is put into the facility. Further, with a view to reuse, a hardly-decomposable auxiliary material is daringly used, in some cases. Thus, it is preferable to subject the raw material after being subjected to the first drying substep, to sieving to remove the remaining auxiliary material.

### [Pulverization Step]

The raw material dried after being subjected to the second drying substep to have a water content falling within the second rage can be non-uniform in terms of particle size. In this situation, the raw material is preferably pulverized to have an arbitrary particle size, as necessary. Through pulverization, the particle size of the raw material is uniformed, so that it becomes possible to facilitate a processing of the seeding agent to be optionally performed in a subsequent step

### [Compacting Step]

As above, through the optional pulverization step after completion of the drying step, a pellet-shaped seeding agent can be obtained. Although the pellet-shaped seeding agent can sufficiently function in this form, it has the following disadvantage, possibly leading to a problem. Assume that the pellet-shaped seeding agent is directly put into a treatment tank of a wastewater treatment facility. In this case, if a large volume of wastewater to be treated flows into the treatment tank within a short period of time, the pellet-shaped seeding agent is likely to be discharged outside the tank without sufficient contact with the wastewater inside the tank, resulting in causing deterioration in effect of the seeding agent. Thus, the pellet-shaped seeding agent is preferably compacted into a block in some way.

For example, as a method for compacting the seeding agent according to the present invention into a block, the technique described in the Patent Document 4 proposed by the inventors of the application may be employed. The technique described in the Patent Document 4 comprises the following steps. First of all, a given amount of binder is added to the pellet-shaped seeding agent. As the binder, it is possible to use, for example, rice bran, rice powder, starch and wheat flour. Then, water is added to a kneaded mixture of the seeding agent and the binder. Respective amounts of the binder and water are appropriately selected depending on an amount of water contained in the seeding agent. For example, the binder is preferably added in an amount of about 5 to 10 weight%, with respect to 100 weight% of the seeding agent, and water is preferably added in an amount of about 25 to 27.5 weight%, with respect to 100 weight% of the mixture of the seeding agent and the binder. Preferably, when water is added, it is added in plural additions, e.g., 15 to 30 additions, and, every time water is added, the seeding agent and the added water are homogenously kneaded.

Then, the seeding agent with the binder and water added thereto is put into a mold and compressed by a given pressure. The compression pressure is preferably about 15 to 50 kg/cm². In this manner, a compacted, block-shaped seeding agent which has high breakage resistance, , uniform hardness, high mold resistance, and a hardness enough to be dissolved after about 120 to 170 hours from input into a treatment tank.

### EXAMPLES

### (Production of Seeding Agent)

As the raw material, a mixture of dewatered sludge, cow dung, a seed material and wood chips was used. The dewatered sludge, the cow dung, the seed material and the wood chips were mixed, respectively, in an amount of 50 weight%, in an amount of 20 weight%, in an amount of 10 weight% and in an amount of 20 weight%. A water content of the raw material was 65 weight%. As the seed material, compost prepared by fermenting organic waste was used. Although a plurality of types of bacteria and actinomycetes were mixed in the seed material, theses were not mixed as a microbial group having a specific feature under intentional control. The raw material was mixed using a manual spreader, and the mixed raw material was put into a fermenter. The fermenter had a size of 4.9 m length × 3.8 m width × 3.0 m height, and a structure in which five air blast pipes were buriedly inserted into a bottom wall thereof, and 1.5t of the raw material was loaded into the fermenter.

A temperature of the above raw material was increased until it rose beyond 70°C, under adjustment of an air supply amount from the air blast pipes. After the temperature rose beyond 70°C, the air supply amount was reduced, and, in this state, fermentation was maintained. Subsequently, in summer, when the temperature of the raw material fell below 70°C, or when about one week had elapsed under the condition that the temperature of the raw material was maintained at 70°C, or, in winter, when the temperature of the raw material fell below 70°C, or when about 10 to 14 days had elapsed under the condition that the temperature of the raw material was maintained at 70°C, plowing was performed by transferring the raw material to another fermenter having the same size and structure. After the plowing, the temperature of the raw material was increased until it rose beyond 70°C again, under adjustment of the air supply amount, and the second plowing was performed at the same timing as that described above. The above process is equivalent to the first fermentation substep.

After the first fermentation substep, i.e., after the second plowing, the temperature of the raw material was increased until it rose beyond 50°C, under adjustment of the air supply amount, and, in this state, the fermentation was maintained. In summer, when the temperature of the raw material fell below 50°C, or when about one week had elapsed under the condition that the temperature of the raw material was maintained at 50°C, or, in winter, when the temperature of the raw material fell below 50°C, or when about 10 to 14 days had elapsed under the condition that the temperature of the raw material was maintained at 50°C, the fermentation step was terminated. This above process is equivalent to the second fermentation substep. At this point, a weight of the raw material was about 9 t.

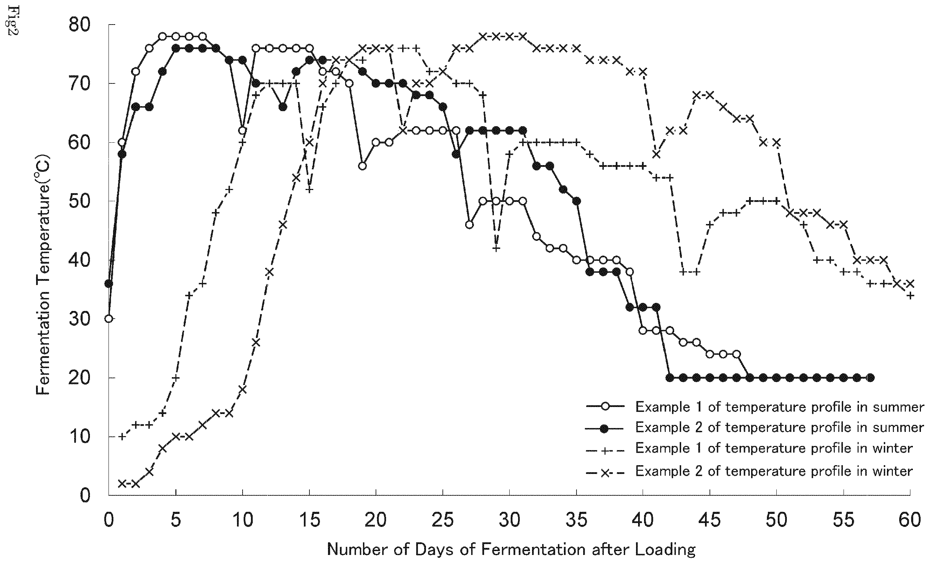

FIG. 2 illustrates typical temperature profiles in summer and winter when the raw material is actually fermented under the above fermentation control.

Subsequently, the raw material after being subjected to the fermentation step was gradually dried. At this point, the water content thereof was 57 to 59 weight%. The water content of the raw material was reduced to about 50% or less by supplying air at normal temperature to the raw material at a flow rate of 1.0 to 5.0 m³/sec for 6 to 9 hours/day over about 4 weeks, and performing plowing. During this process, as needed, a part of the raw material was subjected to natural drying in the sum, and returned to the original raw material to thereby promote vaporization of water. The above process is equivalent to the first drying substep.

Subsequently, the raw material having a water content reduced to 50% or less was screened using a sieve having a sieve mesh of 10 mm to remove wood chips which had not been fully decomposed. The weight of the raw material after the removal of remaining wood chips was about 5 t.

The sieved raw material (i.e., the raw material after the removal of remaining wood chips) was dried by hot air at 50°C from a flat-type drying device, until the water content thereof was reduced to 20% or less. A period of time required for the drying was 24 to 72 hours.

### (Analysis Result of Microorganisms contained in Seeding Agent)

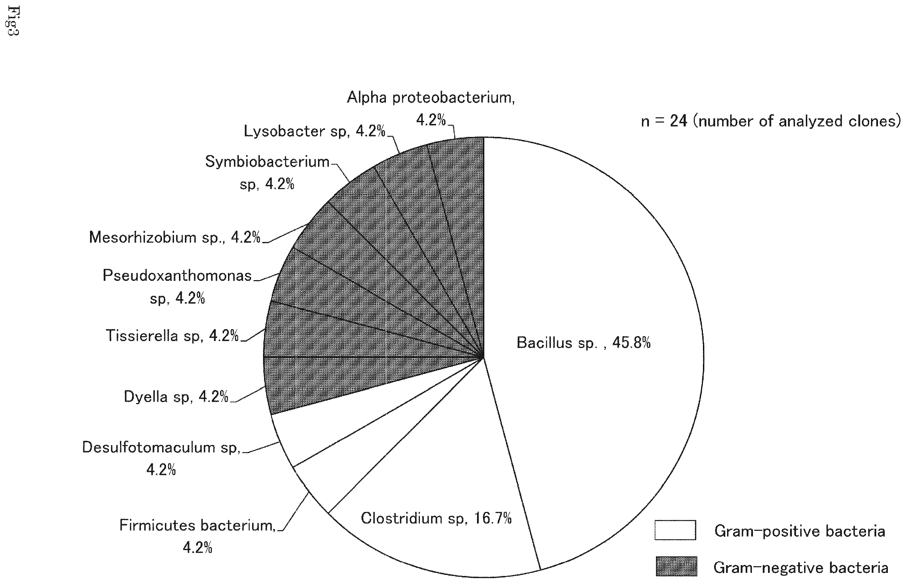

FIG. 3 presents a result obtained by analyzing a type of microorganism contained in the seeding agent produced in the above manner. The analysis of the type of microorganism was performed with a focus on bacteria contained therein and using 16S rDNA clone library analysis. A clone library was constructed by incorporating into, a plasmid vector, a region corresponding to 9F-1451R (PCA primer 9F: GAGTTTGATCCTGGCTCAG, and 1541R: AAGGAGGTGATCCAGC) of 16S rDNA. The type of microorganism was estimated based on information about a sequence of about 300 to 500 bp from 9F of the 16S rDNA fragments and by utilizing the BLAST search system provided by DDBJ (DDBJ: http://blast.ddbj.nig.ac.jp/blast/blastn?lang=ja). FIG. 3 shows that the seeding agent according to the present invention contains 4 genera of Gram-positive bacteria in a total percentage of 71.8%.

### (Analysis Result of Difference in Type of Microorganism depending on Drying Step)

The feature of the present invention is in that a drying step of drying the raw material after being subjected to the fermentation step is divided into two substeps, wherein the raw material is gradually dried to reach a given range of water content by taking a relatively long period of time, and then rapidly dried to reach a given range of water content within a shorter period of time. In order to verify an advantageous effect of the feature of the present invention, it was analyzed what kind of difference in type of microorganism contained in the seeding agent appears depending on a drying step. Each of 4 samples at the following 4 time points (A to D) after the fermentation was subjected to shake culture in an artificial wastewater culture medium at 20°C for 24 hours, to analyze a type of bacterium. That is, a difference in type of bacterium emerging when using each of the 4 samples as a seeding agent was ascertained. The analysis method for the type of bacterium is the same as that in FIG. 3. The sample D is equivalent to the seeding agent produced by the production method according to the present invention.
(A) A sample at a time of completion of fermentation (water content: 59%)
(B) A sample obtained by drying the sample A at the time of completion of fermentation, until the water content becomes 50% or less, by taking about 2 weeks
(C) A sample obtained by drying the sample A at the time of completion of fermentation, until the water content becomes 20% or less, at 50°C by taking 24 hours
(D) A sample obtained by drying the sample B until the water content becomes 20% or less, at 50°C by taking 24 hours

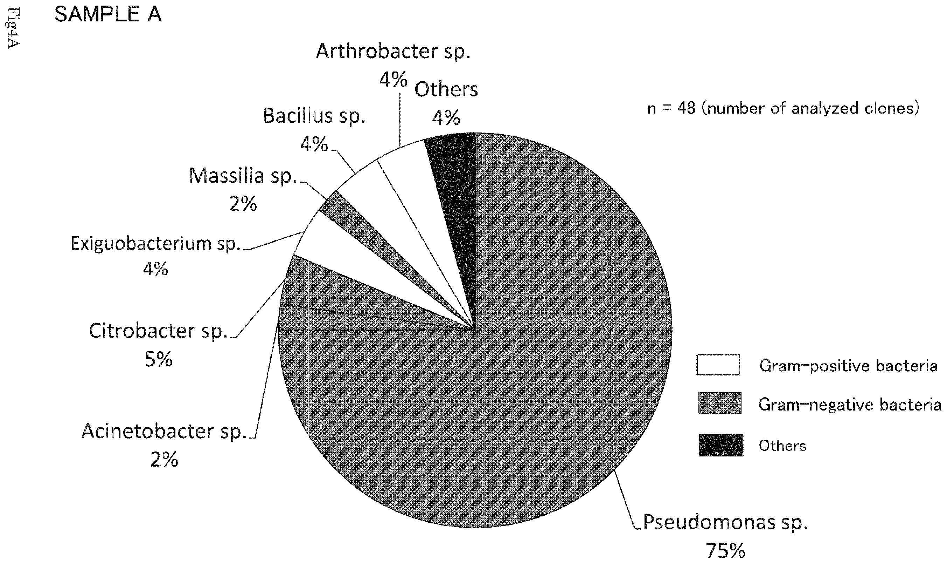

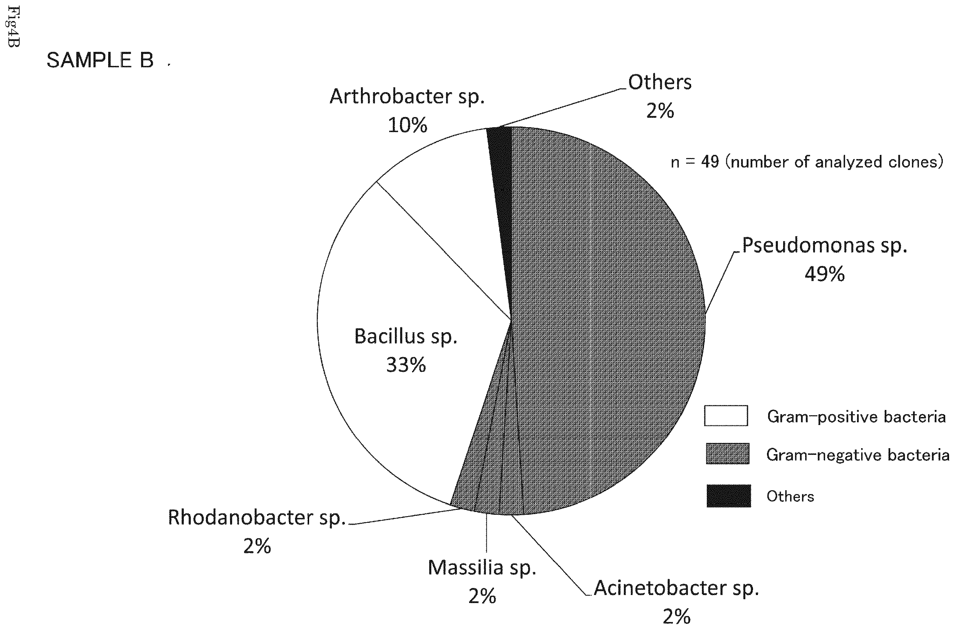

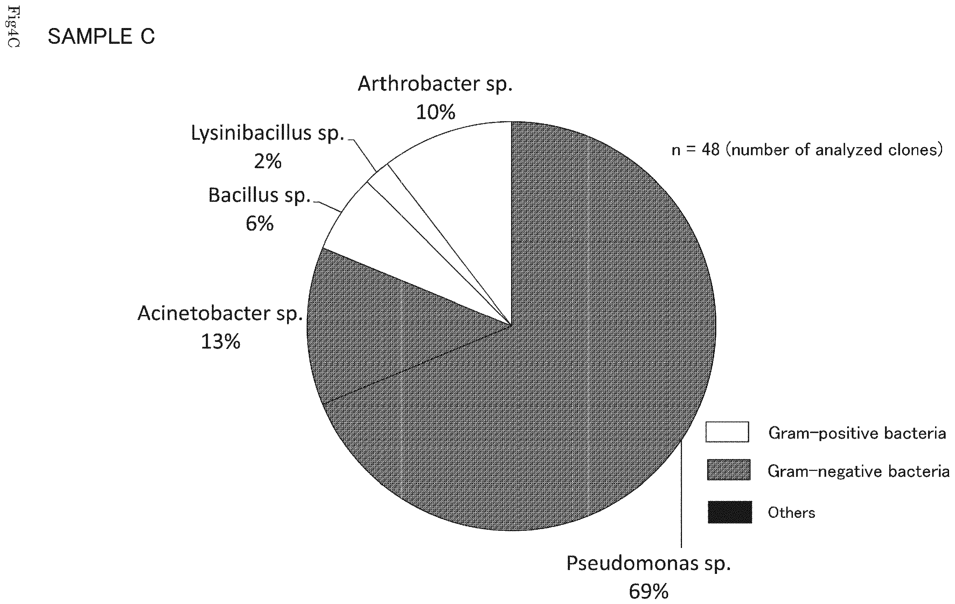

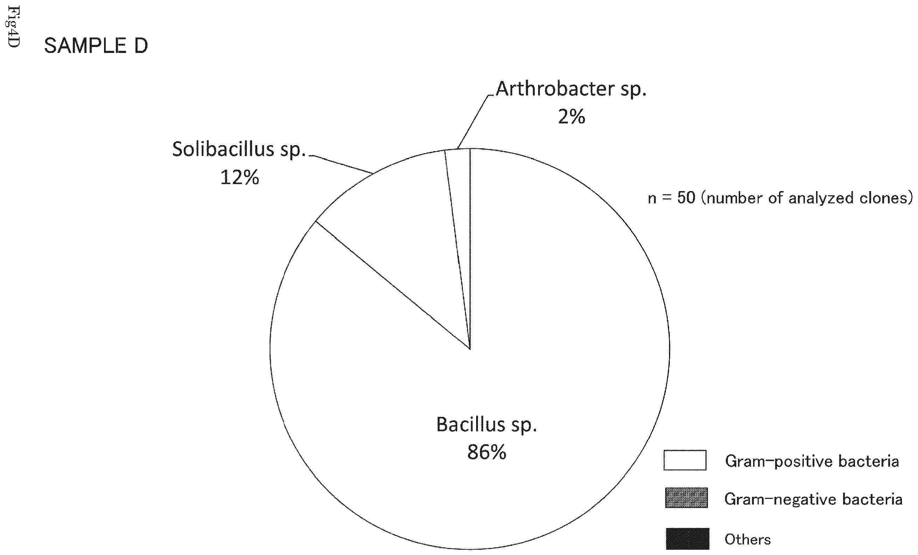

FIGS. 4A to 4D illustrate analysis results of a type of bacterium emerging when using, as the seeding agent, each of the samples A to D. In the sample A, Gram-negative bacteria account for 84% of the total, and Gram-positive bacteria account for only 12%. In the sample B equivalent to a sample obtained by gradually drying the sample A, Gram-positive bacteria dominantly emerge, and account for 43% of the total. In the sample D, i.e., a sample obtained by reducing the water content just after completion of fermentation, to 50% or less by taking about 2 weeks (equivalent to the first drying substep as set forth in the appended claims), and then further reducing the water content to 20% or less, by taking 24 hours (equivalent to the second drying substep as set forth in the appended claims), Gram-positive bacteria dominantly emerged, and no Gram-negative bacterium emerged. This can be deemed as a result of the phenomenon that, in the second drying substep, unwanted bacteria other than bacteria sporulated in the first substep are decomposed. On the other hand, in the sample C, i.e., a sample obtained by reducing the water content just after completion of fermentation, to 20% or less by taking 24 hours, without going through the first drying substep as set forth in the appended claims, a content rate of Bacillus bacteria was significantly small (6%), and a quite large number of Pseudomonas bacteria emerged (69%). These results show that, for producing a seeding agent containing a variety of and a large amount of Gram-positive bacteria as effective microorganisms effectively functioning in wastewater treatment facilities, the production method comprising the first drying substep of gradually drying the fermented raw material by taking a relatively long period of time, and the second drying substep of rapidly drying the resulting raw material is significantly effective.

### (Analysis Result of Biodiversity in Bacterial Group)

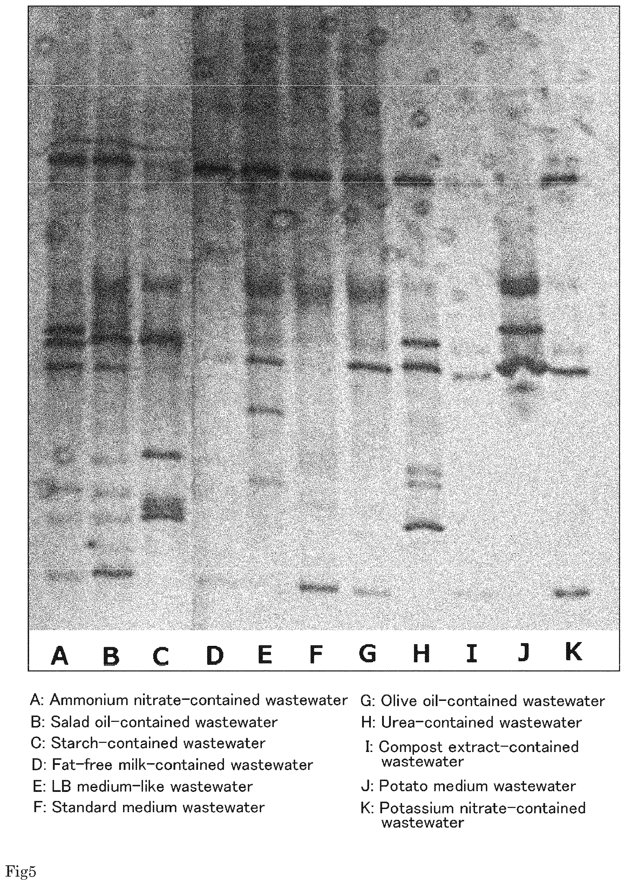

It was analyzed what kind of difference appears in a bacterial group emerging when the seeding agent produced by the production method according to the present invention is put into wastewater, depending on components contained in the wastewater. A suspension liquid containing 1% of n the seeding agent produced by the production method according to the present invention (with respect to 100% of sterile distilled water) was put into each of 11 types A to J of artificial wastewater presented in FIG. 5, in an amount of 1/10 of an amount of the wastewater, and subjected to shake culture in a conical flask at 27°C at 120 rpm, for 5 days. After cultivation, an analysis sample for a bacterial group was collected from a culture solution.

An analysis of a type of microorganism emerging in the seeding agent was performed with a focus on bacteria in the sample and using a denaturing gradient gel electrophoresis method (PCR-DGGE method: gradient of acrylamide gel: 8 → 10%, concentration gradient of denaturant: 20 → 50%, migration: 0.5 × TAE, 200V, 3.5 h), while targeting V3 region of 13S rDNA of a bacterium. As a PCA primer, GC-341f (5'-GC clamp-CCTAGGGAGGCAGCAG-3') and 517r (5'-ATTACCGCGGCTGCTGG-3') was used.

As a result of the analysis, as presented in FIG. 5, it was observed that a bacterial group emerging varies depending on a type of sewage. This shows that the seeding agent of the present invention is high in microbial biodiversity and applicable to a plurality of wastewater treatment facilities placed under various wastewater conditions.

## Claims

1. A method for producing a seeding agent, comprising:
a fermentation step of producing a fermented raw material by aerobically fermenting a raw material comprising organic waste and an auxiliary material, a percentage of water content of the fermented raw material as measured just after completion of the fermentation step is higher than a first range; and
a drying step; **characterized in that**:
the drying step includes a first drying substep and a second drying substep; wherein the first drying substep and the second drying substep are:
the first drying substep of producing a dried raw material to dry the fermented raw material until a percentage of the water content of the fermented raw material is within the first range
the second drying substep to further dry the dried raw material until a percentage of water content of the dried raw material is within a range that is less than a lower limit of the first range, the first drying substep occurs before the second drying substep and is of a duration that is longer than the second drying substep; and
wherein the first range is from 40% to 55%, and the range that is less than the lower limit of the first range is from 10% to 30%.

2. The method as recited in claim 1, wherein
the first drying substep includes drying the fermented raw material after being subjected to the fermentation step, until the water content thereof becomes 50% or less, and
the second drying substep includes further drying the dried raw material after being subjected to the first drying substep, until the water content thereof becomes 20% or less.

3. The method as recited in claim 1 or claim 2, wherein the first drying substep includes sporulating microorganisms contained in the fermented raw material.

4. The method as recited in claim 1 or claim 2, wherein the second drying substep includes decomposing microorganisms other than the microorganisms sporulated in the first drying substep.

5. The method as recited in claim 1 or claim 2, wherein the fermentation step comprises:
a first fermentation substep of increasing a fermentation temperature of the raw material to a temperature of 70°C or more, at least two times; and
a second fermentation substep of increasing a fermentation temperature of the raw material after being subjected to the first fermentation substep, to a temperature of 50°C or more, at least one time.

6. The method as recited in claim 5, wherein at least the second fermentation substep includes a sub-substep of plowing the raw material, and wherein, when the temperature of the raw material in the sub-substep of plowing does not rise beyond 50°C any more, the fermentation step is terminated.

7. The method as recited in claim 1 or claim 2, which comprises a step of, before the second drying substep, removing a remaining part of the auxiliary material from the raw material after being subjected to the first drying substep.

8. The method as recited in claim 1 or claim 2, wherein the organic waste is one selected from the group consisting of sewage sludge, livestock excrement, and food waste, or a combination of two or more thereof.

## Patentansprüche

1. Verfahren zum Herstellen eines Impfungsmittels, das Folgendes umfasst:
einen Fermentationsschritt eines Herstellens eines fermentierten Rohstoffs durch aerobes Fermentieren eines Rohstoffs, der organischen Abfall und einen Hilfsstoff umfasst, wobei ein prozentualer Anteil eines Wassergehalts des fermentierten Rohstoffs, wie unmittelbar nach Abschluss des Fermentationsschritts gemessen, höher als ein erster Bereich ist; und
einen Trocknungsschritt; **dadurch gekennzeichnet, dass**:
der Trocknungsschritt einen ersten Trocknungsunterschritt und einen zweiten Trocknungsunterschritt beinhaltet; wobei der erste Trocknungsunterschritt und der zweite Trocknungsunterschritt Folgende sind:
der erste Trocknungsunterschritt des Herstellens eines getrockneten Rohstoffs, um den fermentierten Rohstoff zu trocknen, bis ein prozentualer Anteil des Wassergehalts des fermentierten Rohstoffs innerhalb des ersten Bereichs liegt
der zweite Trocknungsunterschritt, um den getrockneten Rohstoff weiter zu trocknen, bis ein prozentualer Anteil des Wassergehalts des getrockneten Rohstoffs innerhalb eines Bereichs liegt, der kleiner als eine untere Grenze des ersten Bereichs ist, wobei der erste Trocknungsunterschritt vor dem zweiten Trocknungsunterschritt erfolgt und von einer Dauer ist, die länger als der zweite Trocknungsunterschritt ist; und
wobei der erste Bereich von 40 % bis 55 % reicht und der Bereich, der kleiner als die untere Grenze des ersten Bereichs ist, von 10 % bis 30 % reicht.

2. Verfahren nach Anspruch 1, wobei
der erste Trocknungsunterschritt das Trocknen des fermentierten Rohstoffs beinhaltet, nachdem er dem Fermentationsschritt unterzogen wurde, bis der Wassergehalt davon höchstens 50 % wird, und
der zweite Trocknungsunterschritt das weitere Trocknen des getrockneten Rohstoffs beinhaltet, nachdem er dem ersten Trocknungsunterschritt unterzogen wurde, bis der Wassergehalt davon höchstens 20 % wird.

3. Verfahren nach Anspruch 1 oder 2, wobei der erste Trocknungsunterschritt sporulierende Mikroorganismen beinhaltet, die in dem fermentierten Rohstoff enthalten sind.

4. Verfahren nach Anspruch 1 oder 2, wobei der zweite Trocknungsunterschritt ein Zersetzen von anderen Mikroorganismen als den in dem ersten Trocknungsunterschritt sporulierten Mikroorganismen beinhaltet.

5. Verfahren nach Anspruch 1 oder 2, wobei der Fermentationsschritt Folgendes umfasst:
einen ersten Fermentationsunterschritt eines Erhöhens einer Fermentationstemperatur des Rohstoffs wenigstens zwei Mal auf eine Temperatur von wenigstens 70 °C; und
einen zweiten Fermentationsunterschritt des Erhöhens einer Fermentationstemperatur des Rohstoffs, nachdem er dem ersten Fermentationsunterschritt unterzogen wurde, wenigstens ein Mal auf eine Temperatur von wenigstens 50 °C.

6. Verfahren nach Anspruch 5, wobei wenigstens der zweite Fermentationsunterschritt einen Unterunterschritt eines Rührens des Rohstoffs beinhaltet und wobei, wenn die Temperatur des Rohstoffs in dem Unterunterschritt des Rührens nicht mehr über 50 °C ansteigt, der Fermentationsschritt beendet wird.

7. Verfahren nach Anspruch 1 oder 2, das einen Schritt, vor dem zweiten Trocknungsunterschritt, eines Entfernens eines verbleibenden Teils des Hilfsstoffs aus dem Rohstoff umfasst, nachdem er dem ersten Trocknungsunterschritt unterzogen wurde.

8. Verfahren nach Anspruch 1 oder 2, wobei der organische Abfall aus der Gruppe ausgewählt ist, die aus Klärschlamm, Viehkot und Lebensmittelabfall oder einer Kombination von zwei oder mehr davon besteht.

## Revendications

1. Procédé de production d'un agent d'ensemencement, comprenant :
une étape de fermentation consistant à produire une matière première fermentée par fermentation aérobie d'une matière première comprenant des déchets organiques et un matériau auxiliaire, un pourcentage de la teneur en eau de la matière première fermentée tel que mesuré juste après l'achèvement de l'étape de fermentation étant supérieur à une première plage ; et
une étape de séchage ; **caractérisé en ce que** :
l'étape de séchage comporte une première sous-étape de séchage et une seconde sous-étape de séchage ; la première sous-étape de séchage et la seconde sous-étape de séchage étant :
la première sous-étape de séchage de production d'une matière première séchée pour sécher la matière première fermentée jusqu'à ce qu'un pourcentage de la teneur en eau de la matière première fermentée se situe dans la première plage
la seconde sous-étape de séchage pour sécher davantage la matière première séchée jusqu'à ce qu'un pourcentage de la teneur en eau de la matière première séchée se situe dans une plage qui est en-dessous d'une limite inférieure de la première plage, la première sous-étape de séchage se produisant avant la seconde sous-étape de séchage et étant d'une durée plus longue que la seconde sous-étape de séchage ; et
la première plage est de 40 % à 55 %, et la plage qui est en-dessous de la limite inférieure de la première plage est de 10 % à 30 %.

2. Procédé selon la revendication 1, dans lequel
la première sous-étape de séchage comporte le séchage de la matière première fermentée après avoir été soumise à l'étape de fermentation, jusqu'à ce que sa teneur en eau devienne inférieure ou égale à 50 %, et
la seconde sous-étape de séchage comporte le séchage supplémentaire de la matière première séchée après avoir été soumise à la première sous-étape de séchage, jusqu'à ce que sa teneur en eau devienne inférieure ou égale à 20 %.

3. Procédé selon la revendication 1 ou la revendication 2, dans lequel la première sous-étape de séchage comporte des microorganismes sporulants contenus dans la matière première fermentée.

4. Procédé selon la revendication 1 ou la revendication 2, dans lequel la seconde sous-étape de séchage comporte des microorganismes de décomposition autres que les microorganismes sporulés dans la première sous-étape de séchage.

5. Procédé selon la revendication 1 ou la revendication 2, dans lequel l'étape de fermentation comprend :
une première sous-étape de fermentation d'augmentation d'une température de fermentation de la matière première à une température de 70 °C ou plus, au moins deux fois ; et
une seconde sous-étape de fermentation d'augmentation d'une température de fermentation de la matière première après avoir été soumise à la première sous-étape de fermentation, à une température de 50 °C ou plus, au moins une fois.

6. Procédé selon la revendication 5, dans lequel au moins la seconde sous-étape de fermentation comporte une sous-sous-étape de labour de la matière première, et dans lequel, lorsque la température de la matière première dans la sous-sous-étape de labour ne dépasse plus 50 °C, l'étape de fermentation est terminée.

7. Procédé selon la revendication 1 ou la revendication 2, qui comprend une étape, avant la seconde sous-étape de séchage, d'élimination d'une partie restante de la matière auxiliaire de la matière première après avoir été soumise à la première sous-étape de séchage.

8. Procédé selon la revendication 1 ou la revendication 2, dans lequel les déchets organiques sont choisis dans le groupe constitué par les boues d'épuration, les excréments de bétail et les déchets alimentaires, ou une combinaison d'au moins deux de ceux-ci.
